(19) Europäisches Patentamt
European Patent Office
Office européen des brevets

(11) Publication number: **0 181 284**
**B1**

(12) **EUROPEAN PATENT SPECIFICATION**

(45) Date of publication of patent specification: **07.02.90**

(21) Application number: **85810493.8**

(22) Date of filing: **29.10.85**

(51) Int. Cl.$^5$: **C 07 C 229/42**, C 07 C 227/18, C 07 C 255/33, C 07 C 255/50, C 07 C 327/26, A 01 N 37/44

# (54) Amino acid esters.

(30) Priority: **05.11.84 US 668056**

(43) Date of publication of application:
**14.05.86 Bulletin 86/20**

(45) Publication of the grant of the patent:
**07.02.90 Bulletin 90/06**

(84) Designated Contracting States:
**AT BE CH DE FR GB IT LI NL**

(56) References cited:
**DE-A-2 812 169**

The file contains technical information submitted after the application was filed and not included in this specification

(73) Proprietor: **SANDOZ AG**
**Lichtstrasse 35**
**CH-4002 Basel (CH)**
(84) **BE CH FR GB IT LI NL**

(73) Proprietor: **SANDOZ-PATENT-GMBH**
**Humboldtstrasse 3**
**D-7850 Lörrach (DE)**
(84) **DE**

(73) Proprietor: **SANDOZ-ERFINDUNGEN Verwaltungsgesellschaft m.b.H.**
**Brunner Strasse 59**
**A-1235 Wien (AT)**
(84) **AT**

(72) Inventor: **Henrick, Clive A.**
**3177 Manchester Ct.**
**Palo Alto California 94303 (US)**
Inventor: **Garcia, Barbara A.**
**452 Carmelita Drive**
**Mountain View California 94040 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 181 284 B1

**Description**

This invention relates to novel esters and *S*-thioesters of amino acids, intermediates therefor, synthesis thereof and the use of said esters and thioesters for the control of pests.

More particularly, the compounds of the present invention are represented by the following generic formula (A):

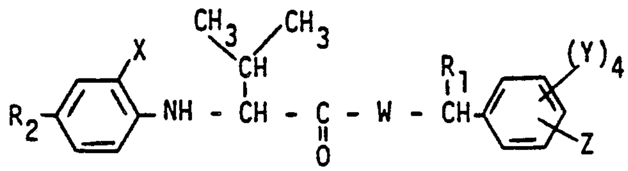

(A)

wherein

$R_1$ is hydrogen, methyl, ethynyl or cyano;

$R_2$ is lower alkyl, lower haloalkyl, lower alkoxy, lower haloalkoxy or halogen;

W is oxygen or sulfur;

X is hydrogen, chloro or fluoro;

Y is halogen;

Z is H, lower alkyl, lower haloalkyl, lower alkoxy, lower alkylcarbonyl, lower alkoxycarbonyl, nitro, cyano, halogen, lower alkenyl or lower alkoxyalkyl.

The compounds of the present invention of formula (A) can have one or more asymmetric carbon atoms. The present invention includes each of the optical isomers and racemic mixtures thereof, such as the (*R*)-acid, (*R,S*)-alcohol diastereomeric pair of formula (A). In the examples hereinafter, unless otherwise specified, the compound is a racemic mixture.

The following terms, wherever used in the description herein and in the appended claims, have the meaning defined below, unless otherwise specified hereinafter.

The term "lower alkyl" refers to an alkyl group, straight or branched, having a chain length of one to eight particularly 1 to 5 carbon atoms and refers preferably to methyl. The term "lower haloalkyl" refers to a lower alkyl group substituted with one to six halogen atoms. A preferred lower haloalkyl significance is $CF_3$.

The term lower alkoxyalkyl refers to an alkyl group substituted at one of its carbon atoms by an alkoxy group (e.g. as defined below), the total number of carbon atoms being not greater than ten.

The term "lower alkenyl" refers to an ethylenically unsaturated hydrocarbon group, straight or branched having a chain length of up to 8 carbon atoms and 1 or 2 ethylenic bonds. Preferred lower alkenyl significances have 1 ethylenic bond and 3 to 8 carbon atoms. The allyl group is a particularly preferred lower alkenyl.

The term "lower alkoxy" refers to an alkoxy group, straight or branched, having a chain length of one to eight, particularly 1 to 5 carbon atoms and refers preferably to $OCH_3$. The term "lower haloalkoxy" refers to a lower alkoxy group substituted with one to six halogen atoms.

The term "lower alkylcarbonyl" refers to an alkylcarbonyl group, straight or branched, having a chain length of two to eight carbon atoms, such as methylcarbonyl.

The term "lower alkoxycarbonyl" refers to an alkoxycarbonyl group, straight or branched, having a chain length of two to eight carbon atoms, such as methoxycarbonyl.

The compounds of the invention of formula (A) can be prepared by conventional esterification processes.

Such processes involve esterifying a compound of formula I,

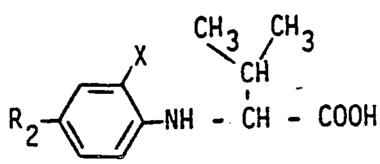

I

wherein X and $R_2$ are as defined above, or a reactive functional derivative thereof, with a compound of formula II

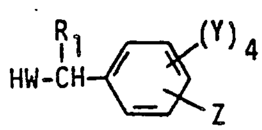

II

wherein W, Y, Z and $R_1$ are as defined above, or a reactive functional derivative thereof.

Suitable reactive functional derivatives of compounds of formula I are for example the corresponding acid halides (e.g. acid chlorides or acid bromides) or salts (e.g. alkali metal carboxylates) of compounds of formula I.

Suitable reactive functional derivaties of compounds of formula II are the corresponding benzyl halides.

Where compound of formula I is reacted in acid form, the esterification process is conveniently effected in the presence of an acid catalyst; where compound of formula I is reacted in acid halide form, the esterification process is conveniently effected in the presence of the base.

The esterification is conveniently effected in a solvent which is inert under the reaction conditions, such as tetrahydrofuran or methylene chloride.

Individual isomers may be prepared in the same manner but starting with the individual optically pure isomers of compounds of formula I or reactive functional derivatives thereof.

The compounds of formula (A) may be recovered from the reaction mixture in which they are formed by working up by established procedures.

The starting materials and reagents employed in the esterification process of the invention are either known or, insofar as they are not known, may be produced in analogous manner to the processes described herein or to known processes.

The compounds of the present invention of formula (A) are useful pest control agents, particularly for the control of insects, for example those of the order Lepidoptera, Hemiptera, Homoptera, Coleoptera, Diptera, Orthoptera, and Siphonaptera, and other insects, and mites and ticks of the class *Acara*, including mites of the families Tetranychidae and Tarsonemidae and ticks of the families Argasidae and Ixodidae. They are particularly useful for the control of insect pests which inhabit the soil, including Argostis spp., Agriotis spp. and Diabrotica spp. The interesting activity against soil pests is for example illustrated by tests with Diabrotica undecimpunctata involving application of 1 µl of test solutions of 0.5 mg and 1 mg active ingredient in 1 ml acetone. The results indicate useful activity against soil insects, particularly in a corn locus. They also show interesting activity against flies, mosquitoes and Spodoptera spp. and are accordingly indicated for use against public health pests and in a cotton locus. The compounds can be applied to the pest or its locus in a pesticidally effective amount, usually of the order of 0.1 µg to 100 µg per insect, mite or tick, depending on the mode and conditions of application as well as on the pest involved.

Preferred compounds of formula (A) have one or more of the following features:

$R_1$ is preferably H or CN, more preferably H.

$R_2$ is preferably $CF_3$.

W is preferably O.

X is preferably Cl or F, more preferably Cl.

Y is preferably F.

Z is preferably not hydrogen.

Z is preferably in para-position.

Z is preferably $CH_3$, $OCH_3$, $CH_2OCH_3$, allyl, F or $CF_3$, more preferably F or $CH_3$.

The compounds of formula (A) derived from the compounds of formula I (*R*)-configuration are preferred over the corresponding compounds of formula (A) derived from the racemic compounds of formula I.

The compounds of formula (A) are conveniently employed in pest controlling composition form in association with a diluent.

They may be employed in either solid or liquid forms e.g. in the form of a wettable powder or an emulsifiable concentrate incorporating conventional diluents and optionally other formulating agents.

The term diluents as used herein means any liquid or solid agriculturally acceptable material which may be added to the active ingredient(s) to bring it in an easier or improved applicable form, or to a usable or desirable strength of activity. It includes carriers and can for example be talc, kaolin diatomaceous earth, xylene, water and the like.

The compositions of the invention may be produced in conventional manner, e.g. by mixing the active ingredient with a diluent and optionally other formulating ingredients such as surfactants, impregnating agents and the like.

For use as soil insecticides, the compounds of formula (A) are preferably formulated as granules in which the active ingredient is coated on granulated carriers of, for example, corn cobs, gypsum or charcoal.

The compounds of the present invention can be used in combination with other pesticides such as carbamates, phosphates and insect growth regulators, or with insect attractants.

In general, the formulations of the invention include 0.01 to 90% by weight of active agent, from 0 to 20% by weight of agriculturally acceptable surfactant and 99.99% to 10% by weight of diluent.

The following examples are provided to illustrate the practice of the present invention. Temperature is in degrees centigrade. RT means room temperature. Parts are by weight.

Example 1

To a mixture of 2-(2-chloro-4-trifluoromethylanilino)-3-methylbutanoic acid (0.68 g, 2.30 mmol) and potassium carbonate (0.32 g, 2.30 mmol) in 5 ml of tetrahydrofuran (THF) and 1 ml of hexamethylphosphorotribromide (HMPT), at RT and under $N_2$, is added pentafluorobenzyl bromide (0.50 g,

1.92 mmol). The mixture is stirred for 18 hours, after which it is poured into ice water and ether is added. The aqueous phase is extracted with ether (3×), and the combined organic phases are washed with water and with brine and dried. The crude product is purified by preparative thin layer chromatography (prep. TLC) to give pentafluorobenzyl 2-(2-chloro-4-trifluoromethylanilino)-3-methylbutanoate, MS m/e 475 (M+).

nmr ($CDCl_3$) δ = 1.06 [m, 6H, $(CH_3)_2CH$—], 2.22 [m, 1H, $(CH_3)_2CH$—], 3.93 (m, 1H, methine proton adjacent to carbonyl), 5.03 (bm, 1H, N*H*), 5.26 (bs, 2H, methylene protons, 6.56 (bd, 1H, J=8.5Hz, aromatic proton *ortho* to NH), 7.32 (bd, 1H, J=8.5Hz, aromatic proton *para* to Cl) and 7.48 ppm (m, 1H, aromatic proton *ortho* to Cl).

## Example 2

To a mixture of 2-(2-chloro-4-trifluoromethylanilino)-3-methylbutanoic acid (2.59 g, 8.78 mmol), 4-methyl-2,3,5,6-tetrafluorobenzyl alcohol (1.55 g, 7.98 mmol) and a trace amount of dimethylaminopyridine (DMAP) in 80 ml of methylene chloride at approximately 5° is added dicyclohexylcarbodiimide (DCC; 1.65 g, 7.98 mmol) over 3 minutes. The mixture is stirred at 5° for 15 minutes, after which it is warmed slowly to RT and stirred at RT for 1.5 hours. A slight amount of ether is added and the product is filtered. The filtrate is purified by prep. TLC to give 4-methyl-2,3,5,6-tetrafluorobenzyl 2-(2-chloro-4-trifluoromethyl-anilino-3-methylbutanoate, MS m/e 471 ($M^+$).

nmr ($CDCl_3$) δ = 1.05 [2 sets of doublets, J=7Hz and J=1.25Hz, 6H, $(CH_3)_2CH$], 2.20 [m, 1H, $(CH_3)_2CH$], 2.28 (m, 3H, methyl group on aromatic ring), 3.97 (m, 1H, methine proton adjacent to carbonyl), centre at 5.908 (m, 1H, NH), 5.28 (bs, 2H, methylene protons), 6.60 (d, 1H J=8.5Hz aromatic proton *ortho* to NH), 7.37 (m, 1H, aromatic proton *para* to Cl) and 7.47 ppm (m, 1H, aromatic proton *ortho* to Cl).

## Example 3

Following the procedure of Example 2, 4-methyl-2,3,5,6-tetrafluorobenzyl alcohol is reacted with each of 2-(4-trifluoromethylanilino)-3-methylbutanoic acid and 2-(2-fluoro-4-trifluoromethylanilino)-3-methylbutanoic acid to yield, respectively, 4-methyl-2,3,5,6-tetrafluorobenzyl 2-(4-trifluoromethylanilino)-3-methylbutanoate, and 4-methyl-2,3,5,6-tetrafluorobenzyl 2-(2-fluoro-4-trifluoromethylanilino)-3-methylbutanoate.

## Example 4

Following the procedure of Example 1, 2-(2-chloro-4-trifluoromethylanilino)-3-methylbutanoic acid is reacted with each of the halides under column I to give the corresponding ester under column II.

I

1. 4-trifluoromethyl-2,3,5,6-tetrafluorobenzyl bromide
2. 4-methoxycarbonyl-2,3,5,6-tetrafluorobenzyl bromide
3. 4-methylcarbonyl-2,3,5,6-tetrafluorobenzyl bromide
4. 4-cyano-2,3,5,6-tetrafluorobenzyl bromide
5. 4-allyl-2,3,5,6-tetrafluorobenzyl bromide
6. 4-methoxymethyl-2,3,5,6-tetrafluorobenzyl bromide

II

1. 4-trifluoromethyl-2,3,5,6-tetrafluorobenzyl 2-(2-chloro-4-trifluoromethylaniline)-3-methylbutanoate
2. 4-methoxycarbonyl-2,3,5,6-tetrafluorobenzyl 2-(2-chloro-4-trifluoromethylanilino)-3-methylbutanoate
3. 4-methylcarbonyl-2,3,5,6-tetrafluorobenzyl 2-(2-chloro-4-trifluoromethylanilino)-3-methylbutanoate
4. 4-cyano-2,3,5,6-tetrafluorobenzyl 2-(2-chloro-4-trifluoromethylanilino-3-methylbutanoate
5. 4-allyl-2,3,5,6-tetrafluorobenzyl 2-(2-chloro-4-trifluoromethylanilino)-3-methylbutanoate
6. 4-methoxymethyl-2,3,5,6-tetrafluorobenzyl 2-(2-chloro-4-trifluoromethylanilino)-3-methylbutanoate.

## Example 5

Following the procedure of Example 2, 2-(2-chloro-4-trifluoromethylanilino)-3-methylbutanoic acid is reacted with each of α-methyl-4-trifluoromethyl-2,3,5,6-tetrafluorobenzyl alcohol, α-ethynyl-4-trifluoro-methyl-2,3,5,6-tetrafluorobenzyl alcohol and α-cyano-4-trifluoromethyl-2,3,5,6-tetrafluorobenzyl alcohol to give, respectively,

α-methyl-4-trifluoromethyl-2,3,5,6-tetrafluorobenzyl 2-(2-chloro-4-trifluoromethylanilino)-3-methylbutanoate,

α-ethynyl-4-trifluoromethyl-2,3,5,6-tetrafluorobenzyl 2-(2-chloro-4-trifluoromethylanilino-3-methyl-butanoate, and

α-cyano-4-trifluoromethyl-2,3,5,6-tetrafluorobenzyl 2-(2-chloro-4-trifluoromethylanilino-3-methylbutanoate.

## Example 6

Following the procedure of Example 2, (*R*)-2-(2-chloro-4-trifluoromethylanilino)-3-methylbutanoic acid

(0.68 mmol) is reacted with racemic α-cyano-4-trifluoromethyl-2,3,5,6-tetrafluorobenzyl alcohol (0.75 mmol) to yield (*R,S*)-α-cyano-4-trifluoromethyl-2,3,5,6-tetrafluorobenzyl (*R*)-2-(2-chloro-4-trifluoromethylanilino)-3-methylbutanoate (Compound 6.1).

In the same manner, (*R*)-2-(2-chloro-4-trifluoromethylanilino)-3-methylbutanoic acid is reacted with each of α-cyano-4-methyl-2,3,5,6-tetrafluorobenzyl alcohol, 4-methyl-2,3,5,6-tetrafluorobenzyl alcohol, 2,3,4,5,6-pentafluorobenzyl alcohol, 4-trifluoromethyl-2,3,5,6-tetrafluorobenzyl alcohol, 4-allyl-2,3,5,6-tetrafluorobenzylalcohol and 4-methoxy-2,3,5,6-tetrafluorobenzylalcohol to give, respectively,

a) (*R,S*)-2-α-cyano-4-methyl-2,3,5,6-tetrafluorobenzyl (*R*)-2-(2-chloro-4-trifluoromethylanilino)-3-methylbutanoate (Compound 6.2).

b) 4-methyl-2,3,5,6-tetrafluorobenzyl (*R*)-2-(2-chloro-4-trifluoromethylanilino)-3-methylbutanoate (Compound 6.3) with

nmr ($CDCl_3$) δ = 1.03 and 1.06 [2d, 6H, J=7H, ($CH_3$)$_2$CH] centred at 2.28 (m, 3H, J=2Hz, Ar $CH_3$), centred at 3.96 [[m, 1H, J=6Hz and J=9Hz,

$$—HN—C[(CH_3)_2]H—\underset{\underset{O}{\|}}{C}—]],$$

centred at 5.05 (m, 1H, J=9Hz, N*H*), 5.27 (bs, 2H, —O$CH_2$Ar), and centred at 6.53 and 7.38 ppm (m, 3H, aromatic protons) and

MS m/e ($m^+$ + H) = 472.

c) 2,3,4,5,6-pentafluorobenzyl (*R*)-2-(2-chloro-4-trifluoromethylanilino)-3-methylbutanoate (Compound 6.4) having

nmr ($CDCl_3$) δ = 1.04 and 1.07 [2d, 6H, J=7Hz, ($CH_3$)$_2$CH], centred at 2.25 [m, 1H, J=7Hz, ($CH_3$)$_2$C*H*], centred at 3.97 [[m, 1H, J=6Hz and J=9Hz,

$$HN—C[(CH_3)_2CH]\ H—\underset{O}{C}—]],$$

centred at 5.06 (bd, 1H, J=9Hz, N*H*), 5.26 (bs, 2H, O—$CH_2$—Ar) and centred at 6.56 and 7.37 ppm (m, 3H, aromatic protons) and

MS m/e ($m^+$ + H) = 476.

d) 4-trifluoromethyl-2,3,5,6-tetrafluorobenzyl (*R*)-2-(2-chloro-4-trifluoromethylanilino)-3-methylbutanoate (Compound 6.5) having

nmr ($CDCl_3$) δ = 1.03 (d, 3H), 1.10 (d, 3H), 2.20 (m, 1H), 3.95 (dd, 1H), 5.05 (d, broad, 1H), 5.28 (S, 2H), 6.56 (d, 1H), 7.32 (d, 1H) and 7.50 ppm (m, 1H).

e) 4-allyl-2,3,5,6-tetrafluorobenzyl (R)-2-(2-chloro-4-trifluoro-methylanilino)-3-methylbutanoate (Compound 6.6) having

nmr ($CDCl_3$) = centred at 1.04 and 1.08 [2d, 6H, J=7Hz, ($CH_3$CH—], centred at 2.28 [m, 1H, ($CH_3$)$_2$C*H*—], centred at 3.51 [bd, 2H, J=6Hz, Ar$CH_2$CH=$CH_2$) centred at 3.90 and 4.05 [2d, 1H, J=6Hz, N—C*H*[($CH_3$)$_2$CH]—], centred at 5.17 (m, 3H, N*H* and $H_2$C=CH—), 5.28 (bs, 2H, O$CH_2$Ar), centred at 5.93 (m, 1H, $H_2$C=C*H*—), centred at 6.60 and 7.38 (m, 2H, adjacent aromatic protons), and centred at 7.57 ppm (m, 1H, aromatic proton ortho to $CF_3$ and Cl), and

f) 4-methoxy-2,3,5,6-tetrafluorobenzyl (*R*)-2-(2-chloro-4-trifluoromethylanilino)-3-methylbutanoate (Compound 6.7) having

nmr $CDCl_3$ = 1.2 (2d, 6H), 2.3 (m, 1H), 3.8—4.1 (2d, 1H), 4.2 (m, 3H), 5.2 (m, 1H), 5.3 (s, 2H), 6.7 (d, 1H) and 7.5 ppm (m, 2H).

FORMULATION EXAMPLES

F.1. Emulsifiable concentrate

| | |
|---|---|
| Compound of Example 2 | 50.4 parts |
| Emulsifier | 8 parts |
| Trimetylbenzene | 41.6 parts |

F.2. Wettable powder

| | |
|---|---|
| Compound of formula 6.4 | 27.8 parts |
| Calcium silicate | 40.0 parts |
| Pyrophyllite | 27.2 parts |
| Sodium lignosulfonate | 4 parts |
| Sodium dialkylnaphthalene sulfonate | 1 part |

F.3. Granule

| | |
|---|---|
| Compound of Example 6.3 | 5.6 parts |
| Montmorillonite 20/40 mesh | 93.4 parts |
| Dipropylene glycol | 1 part |

**Claims for the Contracting States: BE CH DE FR GB IT LI NL**

1. A compound of the following formula (A)

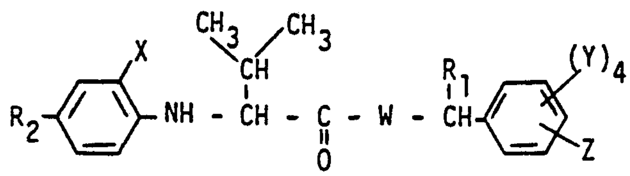

(A)

wherein
$R_1$ is hydrogen, methyl, ethynyl or cyano;
$R_2$ is lower alkyl, lower haloalkyl, lower alkoxy, lower haloalkoxy or halogen;
W is oxygen or sulfur;
X is hydrogen, chloro or fluoro;
Y is halogen;
Z is H, lower alkyl, lower haloalkyl, lower alkoxy, lower alkylcarbonyl, lower alkoxycarbonyl, nitro, cyano, halogen, lower alkenyl or lower alkoxyalkyl.

2. A compound according to Claim 1 wherein $R_1$ is hydrogen or cyano, $R_2$ is trifluoromethyl and W is oxygen.

3. A compound according to Claim 2 wherein Y is fluoro; and Z is H, methyl, trifluoromethyl, methylcarbonyl, methoxycarbonyl, nitro, cyano, chloro, fluoro, allyl, methoxy or methoxymethyl.

4. A compound according to Claim 3 wherein $R_1$ is hydrogen, X is chloro and Z is in the para position.

5. A compound according to Claim 4 wherein Z is $CH_3$, $OCH_3$, $CH_2OCH_3$, allyl, F or $CF_3$.

6. The compound pentafluorobenzyl 2-(2-chloro-4-trifluoromethylanilino)-3-methylbutanoate, according to Claim 5.

7. The compound 4-methyl-2,3,5,6-tetrafluorobenzyl 2-(2-chloro-4-trifluoromethylanilino)-3-methylbutanoate, according to Claim 5.

8. An ester of (*R*)-2-(2-chloro-4-trifluoromethylanilino)-3-methylbutanoic acid according to any one of Claims 4, 5, 6 or 7.

9. Process for the production of a compound of formula (A), stated in any one of Claims 1 to 8, which comprises esterifying a compound of formula I

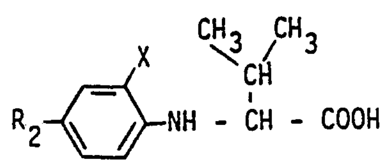

I

wherein X and $R_2$ are as defined in any one of Claims 1 to 8, or a reactive functional derivative thereof, with a compound of formula II

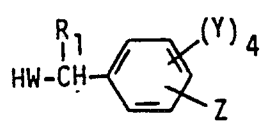

II

wherein $R_1$, W, Y and Z are as defined in any one of Claims 1 to 8, or with a reactive functional derivative thereof.

10. Insecticidal composition comprising a compound of any one of Claims 1 to 8.

11. Method of combatting insects, mites or ticks which comprises applying to the pest or its locus a pesticidally effective amount of a compound according to any one of Claims 1 to 8.

**Claims for the Contracting State: AT**

1. Method of combatting insects, mites or ticks, which comprises applying to the pest or its locus a

pesticidally effective amount of a compound of the following formula (A)

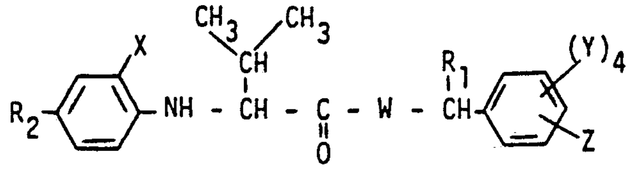

(A)

wherein

$R_1$ is hydrogen, methyl, ethynyl or cyano;

$R_2$ is lower alkyl, lower haloalkyl, lower alkoxy, lower haloalkoxy or halogen;

W is oxygen or sulfur;

X is hydrogen, chloro or fluoro;

Y is halogen;

Z is H, lower alkyl, lower haloalkyl, lower alkoxy, lower alkylcarbonyl, lower alkoxycarbonyl, nitro, cyano, halogen, lower alkenyl or lower alkoxyalkyl.

2. A method according to Claim 1 wherein $R_1$ is hydrogen or cyano, $R_2$ is trifluoromethyl and W is oxygen.

3. A method according to Claim 2 wherein Y is fluoro; and Z is H, methyl, trifluoromethyl, methylcarbonyl, methoxycarbonyl, nitro, cyano, chloro, fluoro, allyl, methoxy or methoxymethyl.

4. A method according to Claim 3 wherein $R_1$ is hydrogen, X is chloro and Z is in the para position.

5. A method according to Claim 4 wherein Z is $CH_3$, $OCH_3$, $CH_2OCH_3$, allyl, F or $CF_3$.

6. A method according to Claim 5, applying pentafluorobenzyl 2-(2-chloro-4-trifluoromethylanilino)-3-methylbutanoate.

7. A method according to Claim 5, applying 4-methyl-2,3,5,6-tetrafluorobenzyl 2-(2-chloro-4-trifluoromethylanilino)-3-methylbutanoate.

8. A method according to any one of Claims 4, 5, 6 or 7 wherein the compound applied is an ester of (*R*)-2-(2-chloro-4-trifluoromethylanilino)-3-methylbutanoic acid.

9. Process for the production of a compound of formula (A), stated in any one of Claims 1 to 8, which comprises esterifying a compound of formula I

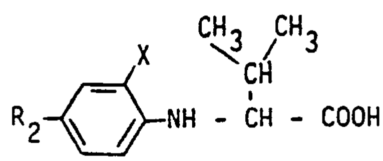

I

wherein X and $R_2$ are as defined in any one of Claims 1 to 8, or a reactive functional derivative thereof, with a compound of formula II

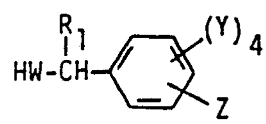

II

wherein $R_1$, W, Y and Z are as defined in any one of Claims 1 to 8, or with a reactive functional derivative thereof.

10. Insecticidal composition comprising a compound as stated in any one of Claims 1 to 8.

**Patentansprüche für die Vertragsstaaten: BE CH DE FR GB IT LI NL**

1. Verbindung der folgenden Formel (A)

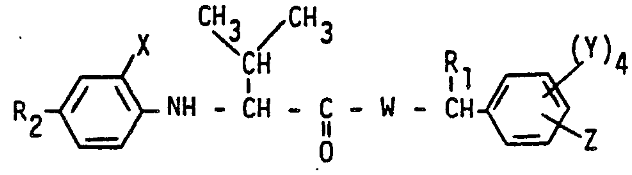

(A)

worin

$R_1$ Wasserstoff, Methyl, Ethinyl oder Cyano ist,

$R_2$ Niederalkyl, Niederhalogenalkyl, Niederalkoxy, Niederhalogenalkoxy oder Halogen bedeutet,
W Sauerstoff oder Schwefel darstellt,
X Wasserstoff, Chlor oder Fluor ist,
Y für Halogen steht und
Z Wasserstoff, Niederalkyl, Niederhalogenalkyl, Niederalkoxy, Niederalkylcarbonyl, Niederalkoxycarbonyl, Nitro, Cyano, Halogen, Niederalkenyl oder Niederalkoxyalkyl bedeutet.

2. Verbindung nach Anspruch 1, dadurch gekennzeichnet, daß $R_1$ Wasserstoff oder Cyano ist, $R_2$ Trifluormethyl bedeutet und W für Sauerstoff steht.

3. Verbindung nach Anspruch 2, dadurch gekennzeichnet, daß Y Fluor ist und Z Wasserstoff, Methyl, Trifluormethyl, Methylcarbonyl, Methoxycarbonyl, Nitro, Cyano, Chlor, Fluor, Allyl, Methoxy oder Methoxymethyl bedeutet.

4. Verbindung nach Anspruch 3, dadurch gekennzeichnet, daß $R_1$ Wasserstoff ist, X Chlor bedeutet und Z in para-Stellung angeordnet ist.

5. Verbindung nach Anspruch 4, dadurch gekennzeichnet, daß Z für $CH_3$, $OCH_3$, $CH_2OCH_3$, Allyl, F oder $CF_3$ steht.

6. Pentafluorbenzyl-2-(2-chlor-4-trifluormethylanilin)-3-methylbutanoat nach Anspruch 5.

7. 4-Methyl-2,3,5,6-tetrafluorbenzyl-2-(2-chlor-4-trifluormethylanilin)-3-methylbutanoat nach Anspruch 5.

8. Ester von (R)-2-(2-Chlor-4-trifluormethylanilin)-3-methylbutansäure nach einem der Ansprüche 4, 5, 6 oder 7.

9. Verfahren zur Herstellung einer Verbindung der Formel (A) nach einem der Ansprüche 1 bis 8, dadurch gekennzeichnet, daß eine Verbindung der Formel I

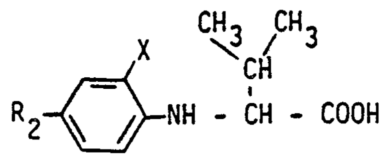

I

worin X und $R_2$ wie in den Ansprüchen 1 bis 8 definiert sind, oder ein reaktionsfähiges funktionelles Derivat hiervon durch Umsetzung mit einer Verbindung der Formel II

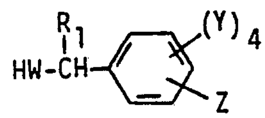

II

worin W, Y, Z und $R_1$ wie in den Ansprüchen 1 bis 8 definiert sind, oder einem reaktionsfähigen funktionellen Derivat hiervon verestert wird.

10. Insektizide Zusammensetzung, dadurch gekennzeichnet, daß sie eine Verbindung nach einem der Ansprüche 1 bis 8 als Wirkstoff enthält.

11. Verfahren zur Bekämpfung von Insekten, Milben oder Zecken, dadurch gekennzeichnet, daß man diese Schädlinge oder ihren Lebensraum mit einer pestizidwirksamen Menge einer Verbindung nach einem der Ansprüche 1 bis 8 als Wirkstoff behandelt.

**Patentansprüche für den Vertragsstaat: AT**

1. Verfahren zur Bekämpfung von Insekten, Milben oder Zecken, dadurch gekennzeichnet, daß man diese Schädlinge oder ihren Lebensraum mit einer pestizidwirksamen Menge einer Verbindung der folgenden Formel (A) behandelt

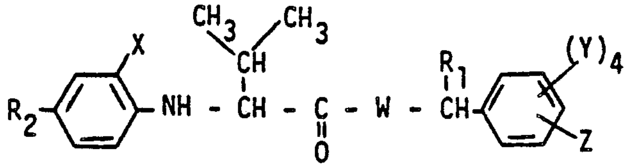

(A)

worin
$R_1$ Wasserstoff, Methyl, Ethinyl oder Cyano ist,
$R_2$ Niederalkyl, Niederhalogenalkyl, Niederalkoxy, Niederhalogenalkoxy oder Halogen bedeutet,
W Sauerstoff oder Schwefel darstellt,
X Wasserstoff, Chlor oder Fluor ist,

Y für Halogen steht und

Z Wasserstoff, Niederalkyl, Niederhalogenalkyl, Niederalkoxy, Niederalkylcarbonyl, Niederalkoxycarbonyl, Nitro, Cyano, Halogen, Niederalkenyl oder Niederalkoxyalkyl bedeutet.

2. Verfahren nach Anspruch 1, dadurch gekennzeichnet, daß $R_1$ Wasserstoff oder Cyano ist, $R_2$ Trifluormethyl bedeutet und W für Sauerstoff steht.

3. Verfahren nach Anspruch 2, dadurch gekennzeichnet, daß Y Fluor ist und Z Wasserstoff, Methyl, Trifluormethyl, Methylcarbonyl, Methoxycarbonyl, Nitro, Cyano, Chlor, Fluor, Allyl, Methoxy oder Methoxymethyl bedeutet.

4. Verfahren nach Anspruch 3, dadurch gekennzeichnet, daß $R_1$ Wasserstoff ist, X Chlor bedeutet und Z in para-Stellung angeordnet ist.

5. Verfahren nach Anspruch 4, dadurch gekennzeichnet, daß Z für $CH_3$, $OCH_3$, $CH_2OCH_3$, Allyl, F oder $CF_3$ steht.

6. Verfahren nach Anspruch 5, dadurch gekennzeichnet, daß Pentafluorbenzyl-2-(2-chlor-4-trifluormethylanilin)-3-methylbutanoat als Wirkstoff verwendet wird.

7. Verfahren nach Anspruch 5, dadurch gekennzeichnet, daß 4-Methyl-2,3,5,6-tetrafluorbenzyl-2-(2-chlor-4-trifluormethylanilin)-3-methylbutanoat als Wirkstoff verwendet wird.

8. Verfahren nach einem der Ansprüche 4, 5, 6 oder 7, dadurch gekennzeichnet, daß ein Ester von (R)-2-(2-Chlor-4-trifluormethylanilin)-3-methylbutansäure als Wirkstoff verwendet wird.

9. Verfahren zur Herstellung einer Verbindung der Formel (A) nach einem der Ansprüche 1 bis 8, dadurch gekennzeichnet, daß eine Verbindung der Formel I

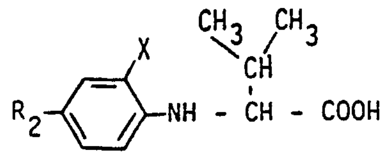

I

worin X und $R_2$ wie in den Ansprüchen 1 bis 8 definiert sind, oder ein reaktionsfähiges funktionelles Derivat hiervon durch Umsetzung mit einer Verbindung der Formel II

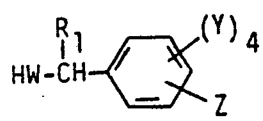

II

worin W, Y, Z und $R_1$ wie in den Ansprüchen 1 bis 8 definiert sind, oder einem reaktionsfähigen funktionellen Derivat hiervon verestert wird.

10. Insektizide Zusammensetzung, dadurch gekennzeichnet, daß sie eine Verbindung nach einem der Ansprüche 1 bis 8 als Wirkstoff enthält.

**Revendications pour les Etats contractants: BE CH DE FR GB IT LI NL**

1. Un composé de formule (A)

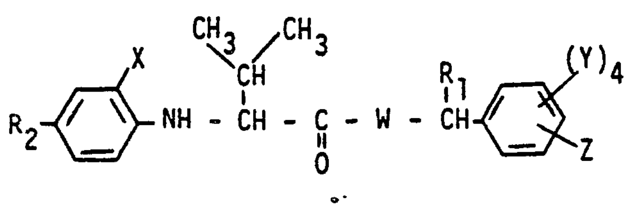

(A)

dans laquelle

$R_1$ signifie l'hydrogène, méthyle, éthynyle ou cyano,

$R_2$ signifie un alkyle inférieur, haloalkyle inférieur, alcoxy inférieur, haloalcoxy inférieur ou un halogène,

W signifie l'oxygène ou le soufre,

X signifie l'hydrogène, chloro ou fluoro,

Y signifie un halogène,

Z signifie H, alkyle inférieur, haloalkyle inférieur, alcoxy inférieur, alkylcarbonyle inférieur, alcoxycarbonyle inférieur, nitro, cyano, un halogène, alcényle inférieur ou alcoxyalkyle inférieur.

2. Un composé selon la revendication 1, dans lequel $R_1$ signifie l'hydrogène ou cyano, $R_2$ signifie trifluorométhyle et W signifie l'oxygène.

3. Un composé selon la revendication 2, dans lequel Y signifie fluoro; et Z signifie H, méthyle, trifluorométhyle, méthylcarbonyle, méthoxycarbonyle, nitro, cyano, chloro, fluoro, allyle, méthoxy ou méthoxyméthyle.

4. Un composé selon la revendication 3, dans lequel $R_1$ signifie l'hydrogène, X signifie chloro et Z est situé en position para.

5. Un composé selon la revendication 4, dans lequel Z signifie $CH_3$, $OCH_3$, $CH_2OCH_3$, allyle, F ou $CF_3$.

6. Le composé 2-(2-chloro-4-trifluorométhylanilino)-3-méthylbutanoate de pentafluorobenzyle, selon la revendication 5.

7. Le composé 2-(2-chloro-4-trifluorométhylanilino)-3-méthylbutanoate de 4-méthyl-2,3,5,6-tétrafluorobenzyle, selon la revendication 5.

8. Un ester de l'acide (*R*)-2-(2-chloro-4-trifluorométhylanilino)-3-méthylbutanoïque selon l'une quelconque des revendications 4, 5, 6 ou 7.

9. Procédé de préparation d'un composé de formule (A) tel que spécifié à l'une quelconque des revendications 1 à 8, qui comprend l'estérification d'un composé de formule I

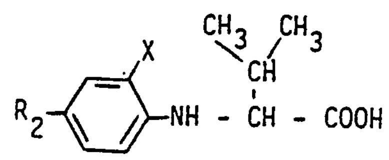

I

dans laquelle X et $R_2$ sont tels que définis à l'une quelconque des revendications 1 à 8, ou d'un dérivé fonctionnel réactif de ce composé, avec un composé de formule II

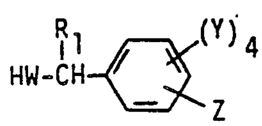

II

dans laquelle $R_1$, W, Y et Z sont tels que définis à l'une quelconque des revendications 1 à 8, ou avec un dérivé fonctionnel réactif de ce composé.

10. Composition insecticide comprenant un composé selon l'une quelconque des revendications 1 à 8.

11. Méthode pour combattre les insectes, les acariens et les tiques, qui comprend l'application sur l'organisme nuisible ou son habitat d'une quantité efficace d'un point de vue pesticide d'un composé selon l'une quelconque des revendications 1 à 8.

**Revendications pour l'Etat contractant: AT**

1. Méthode pour combattre les insectes, les acariens ou les tiques, qui comprend l'application sur l'organisme nuisible ou son habitat d'une quantité efficace d'un point de vue pesticide d'un composé de formule (A)

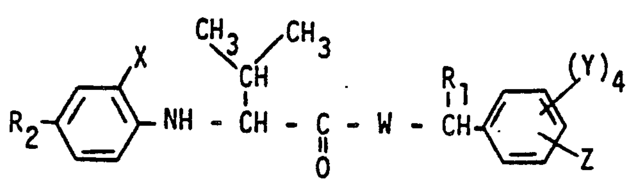

(A)

dans laquelle

$R_1$ signifie l'hydrogène, méthyle, éthynyle ou cyano,

$R_2$ signifie un alkyle inférieur, haloalkyle inférieur, alcoxy inférieur, haloalcoxy inférieur ou un halogène,

W signifie l'oxygène ou le soufre,

X signifie l'hydrogène, chloro ou fluoro,

Y signifie un halogène,

Z signifie H, alkyle inférieur, haloalkyle inférieur, alcoxy inférieur, alkylcarbonyle inférieur, alcoxycarbonyle inférieur, nitro, cyano, un halogène, alcényle inférieur ou alcoxyalkyle inférieur.

2. Une méthode selon la revendication 1, dans laquelle $R_1$ signifie l'hydrogène ou cyano, $R_2$ signifie trifluorométhyle et W signifie l'oxygène.

3. Une méthode selon la revendication 2, dans laquelle Y signifie fluoro; et Z signifie H, méthyle, trifluorométhyle, méthylcarbonyle, méthoxycarbonyle, nitro, cyano, chloro, fluoro, allyle, méthoxy ou méthoxyméthyle.

4. Une méthode selon la revendication 3, dans laquelle $R_1$ signifie l'hydrogène, X signifie chloro et Z est situé en position para.

5. Une méthode selon la revendication 4, dans laquelle Z signifie $CH_3$, $OCH_3$, $CH_2OCH_3$, allyle, F ou $CF_3$.

6. Une méthode selon la revendication 5, caractérisée en ce qu'on applique le 2-(2-chloro-4-trifluorométhylanilino)-3-méthylbutanoate de pentafluorobenzyle.

7. Une méthode selon la revendication 5, caractérisée en ce qu'on applique le 2-(2-chloro-4-trifluorométhylanilino)-3-méthylbutanoate de 4-méthyl-2,3,5,6-tétrafluorobenzyle.

8. Une méthode selon l'une quelconque des revendications 4, 5, 6 ou 7, dans laquelle le composé appliqué est un ester de l'acide (*R*)-2-(2-chloro-4-trifluorométhylanilino)-3-méthylbutanoïque.

9. Procédé de préparation d'un composé de formule (A) tel que spécifié à l'une quelconque des revendications 1 à 8, qui comprend l'estérification d'un composé de formule I

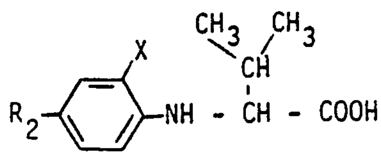

I

dans laquelle X et $R_2$ sont tels que définis à l'une quelconque des revendications 1 à 8, ou d'un dérivé fonctionnel réactif de ce composé, avec un composé de formule II

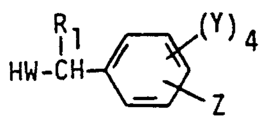

II

dans laquelle $R_1$, W, Y et Z sont tels que définis à l'une quelconque des revendications 1 à 8, ou avec un dérivé fonctionnel réactif de ce composé.

10. Composition insecticide comprenant un composé tel que spécifié à l'une quelconque des revendications 1 à 8.